# EUROPEAN PATENT APPLICATION

(11) **EP 2 336 354 A1**
(43) Date of publication of application: **22.06.2011**
(21) Application number: 10015542.3
(22) Date of filing: 10.12.2010
(51) Int. Cl.: C12Q 1/68

(54) **A method for the detection of a RNA molecule, a kit and a use related thereof**

(30) Priority: 18.12.2009 EP 09015714
(71) Applicant: Roche Diagnostics GmbH, 68305 Mannheim (DE); F.Hoffmann-La Roche AG, 4070 Basel (CH)
(72) Inventor: Albers, Andreas, 82377 Penzberg (DE); Ankenbauer, Waltraud, 82377 Penzberg (DE); Bergmann, Frank, 82393 Iffeldorf (DE); Froehner, Stefanie, 81377 München (DE); Heindl, Dieter, 82396 Paehl (DE)
(74) Representative: Bösl, Raphael Konrad

(57) **Abstract**

In a first aspect, the present invention relates to a method for the detection of a RNA molecule, said method comprising the steps of providing a sample containing the RNA molecule; hybridizing to said RNA molecule a first polynucleotide; extending the first polynucleotide to generate a first strand cDNA; hybridizing a second polynucleotide to the first strand cDNA; extending the first strand cDNA to generate an extension reaction product; amplifying the extension reaction product by means of polymerase chain reaction; and detecting the amplification product by means of real-time fluorescence readout.

In a further aspect, the present invention relates to a kit comprising a first and a second polynucleotide as defined in the present invention, a set of dNTPs, a reverse transcriptase enzyme, and a detection moiety.

In another aspect, the present invention relates to the use of said kit for the detection of an RNA molecule.

SEQUENCE LISTING

<110> Roche Diagnostics GmbH/ F. Hoffmann-La Roche AG

<120> A method for the detection of a RNA molecule, a kit and a use
related thereof

<130> R66746EP2

<160> 11

<170> PatentIn version 3.5

<210> 1
<211> 26
<212> DNA
<213> Artificial Sequence

<220>
<223> mature specific RT-primer (SEQ ID No.: 1)

<400> 1
gccacccacg agccacgcat caacat 26

<210> 2
<211> 26
<212> DNA
<213> Artificial Sequence

<220>
<223> precursor specific RT-primer (SEQ ID No.: 2)

<400> 2
gccacccacg agccacgcat gtcaga 26

<210> 3
<211> 48
<212> RNA
<213> Artificial Sequence

<220>
<223> mi21-dU-prec-helper-UPB (SEQ ID No.: 3)

<400> 3
gccuugccua guccucucag uaauuauauu ugucggguag cuuaucag 48

<210> 4
<211> 19
<212> DNA
<213> Artificial Sequence

<220>
<223> univeral PCR primer A (SEQ ID No.: 4)

<400> 4
gccacccacg agccacgca 19

<210> 5
<211> 20
<212> DNA
<213> Artificial Sequence

<220>
<223> universal PCR primer B (SEQ ID No.: 5)

<400>  5
gccttgccta gtcctctcag                                                    20


<210>  6
<211>  27
<212>  DNA
<213>  Artificial Sequence

<220>
<223>  mi21matu-Probe (SEQ ID No.: 6)


<220>
<221>  misc_binding
<222>  < 1
<223>  /bound moiety="FAM"

<220>
<221>  misc_binding
<222>  > 27
<223>  /bound moiety="BHQ2"

<400>  6
tcgggtagct tatcagactg atgttga                                            27


<210>  7
<211>  27
<212>  DNA
<213>  Artificial Sequence

<220>
<223>  mi21prec-Probe (SEQ ID No.: 7)


<220>
<221>  misc_binding
<222>  < 1
<223>  /bound moiety="HEX"

<220>
<221>  misc_binding
<222>  > 27
<223>  /bound moiety="BHQ2"


<400>  7
cagcccatcg actggtgttg ccatgag                                            27


<210>  8
<211>  39
<212>  DNA
<213>  Artificial Sequence

<220>
<223>  mi21matu-rev-UPA (SEQ ID No.: 8)

<400>  8
gcctccctcg cgccatcagt tttttttttt ttttttcaa                               39


<210>  9
<211>  39
<212>  DNA
<213>  Artificial Sequence

<220>
<223>  mi21prec-rev-UPA (SEQ ID No.: 9)

<400>  9
gcctccctcg cgccatcagt tttttttttt ttttttgtc                              39


<210>  10
<211>  56
<212>  RNA
<213>  Artificial Sequence

<220>
<223>  Mi21-dU-helper-UPB IIg (SEQ ID No.: 10)


<220>
<221>  misc_binding
<222>  > 56
<223>  /bound moiety="phosphate"

<400>  10
gccuugccua guccucucag uaauuauauu ugucggguag cuuaucagac ugaugu          56


<210>  11
<211>  19
<212>  DNA
<213>  Artificial Sequence

<220>
<223>  Universal PCR primer A (SEQ ID No.: 11)

<400>  11
gcctccctcg cgccatcag                                                   19

## Description

In a first aspect, the present invention relates to a method for the detection of a RNA molecule, said method comprising the steps of providing a sample containing the RNA molecule; hybridizing to said RNA molecule a first polynucleotide; extending the first polynucleotide to generate a first strand cDNA; hybridizing a second polynucleotide to the first strand cDNA, characterized in that the second polynucleotide is a 3'-non-extendable oligonucleotide; extending the first strand cDNA to generate an extension reaction product; amplifying the extension reaction product by means of polymerase chain reaction; and detecting the amplification product by means of real-time fluorescence readout. In a further aspect, the present invention relates to a kit comprising a first and a second polynucleotide as defined in the present invention, a set of dNTPs, a reverse transcriptase enzyme, and a detection moiety. In yet another aspect, the present invention relates to the use of the kit according to the present invention for the detection of an RNA molecule.

RNA molecules play an important role in gene expression in a variety of organisms. Recently, small RNAs were discovered as important regulators of post-transcriptional gene expression, in particular gene silencing, with impact on both physiological and pathological processes in living cells. The first gene silencing process guided by small RNAs was discovered in 1993 in the nematode *Caenorhabditis elegans.* There, it was shown that the 21-nts long non-coding RNA transcript of the *lin-4* gene mediates repression of a target gene termed *lin-41.* This repression was shown to depend on complementary base pairing between the short lin-4 RNA molecule and the 3'-untranslated region (UTR) of the mRNA transcript derived from the *lin-41* gene. Since then, short RNAs have been found to be an abundant class of gene regulators in plants, animals, and DNA viruses, and short RNAs of different origin and function have been identified in a variety of organisms from fission yeast to human. Classes of short regulatory RNAs include, e.g., miRNA (microRNA), siRNA (small interfering RNA), piRNA (Piwi-interacting RNA), and rasiRNA (Repeat-associated small interfering RNA). Among them, miRNAs are the most abundant type of small regulatory RNAs in plants and animal. For example, about 3 % of human genes encode for miRNAs, and up to 30 % of human protein coding genes are supposed to be regulated by miRNAs. To date, more than 10.000 different miRNAs have been identified by cloning and sequencing (see, e.g., miRBase: the microRNA registry database at http://www.mirbase.org/, Sanger Institute, UK). In general, miRNAs are characterized by a length of 21-25 nucleotides (nts) and are processed from longer endogenous hairpin transcripts of approximately 70 nucleotides in length, termed precursor-miRNAs or pre-miRNAs. As in the case of lin-4 RNA, miRNAs have been shown to regulate protein expression by a mechanism in which complementary base pairs are formed between the miRNA and its target mRNA. This process leads to the inhibiton of protein translation and, depending on the degree of sequence complementary between the miRNA and its target site, to the degradation of the mRNA transcript (for review see, e.g., Bartel, 2009).

Altered miRNA expression has been implicated to contribute to human disease, in particular to cancer, based on the finding that malignant tumors and tumor cell lines reveal deregulated miRNA expression profiles in comparison to normal tissues (for review see, e.g., Sassen et al., 2008). That is, a global decrease in miRNA levels has been observed in human cancers, indicating that small regulatory RNAs may have an intrinsic function in tumor suppression. Lu et al. (2005) were the first to show that the expression levels of many miRNAs were significantly reduced in cancers compared to the corresponding normal tissues by analyzing a total of 217 human and mouse miRNAs across 334 human cancers, cancer cell lines, and normal tissues. These authors found that cancer goes along with a significantly reduced global miRNA expression, and that poorly differentiated tumors reveal lower miRNA levels in comparison to tumors which were more differentiated. Another recent study examined the expression of 241 human miRNAs in a comprehensive panel of human cancer cell lines, the NCI-60 panel, and in normal tissues, and confirmed the finding that most miRNAs were expressed at lower levels in human tumor-derived cell lines as compared to the corresponding normal tissue (Gaur et al., 2007).

Until recently, uncertainty remained as to whether the altered miRNA expression observed in cancer is a cause or a consequence of malignant transformation. In 2007, a study by Kumar et al. proved for the first time that widespread reduction in miRNA expression does, indeed, promote tumorigenesis. These authors globally reduced the production of mature miRNAs through a knockdown of the miRNA-processing enzymes Drosha and Dicer in cell lines. The mouse and human cancer cells consequently showed decreased steady-state miRNA levels. These cells with global miRNA loss showed enhanced cellular growth in vitro (Kumar et al., 2007). Since there is a global down regulation of miRNAs in tumors, a miRNA profile may reflect the origin and differentiation state of the tumor. Accordingly, the analysis of miRNA expression profiles will soon develop to an important tool in cancer diagnostic, and the application of reliable detection and quantification methods for miRNA levels in individuals will be a prerequisite of highest relevance in this respect.

The detection and quantification of RNA molecules, including the detection of small regulatory RNAs such as miRNAs, can in principle be carried out by a variety of standard procedures, including standard nucleic acid hybridization-based technologies such as Northern hybridisation, RNase protection, primer extension, or microarray hybridization. These methods, however, are not applicable for daily laboratory routine use, since they either rely on the use of radiolabeled agents, and/or are too cost intensive.

Real-time PCR technologies have been developed for the quantification of both precursors and mature miRNAs (see, e.g., Schmittgen et al., 2008). In principle, two methods for amplification and detection of small RNA molecules, including the detection of mature miRNAs, are routinely used. One method is based upon a specific stem-loop RT-primer, containing a universal PCR primer, which is combined with a miRNA-specific PCR primer for the amplification. In this system (see, e.g., Applied Biosystems TaqMan® MicroRNA Assay, Applied Biosystems, USA), target mature miRNAs are detected by hydrolysis probes. The second method uses a universal PCR primer (attached to a specific RT-primer) and a LNA (Locked Nucleic Acid) primer for amplification and SybrGreen® for the detection of target miRNAs (see, e.g., miRCURY LNA™ Univeral RT microRNA PCR, Exiqon, Denmark). The discrimination between mature and precursor forms of miRNA in one sample by these methods, however, is rather difficult.

Hence, there is always a need for an improved method of detecting small RNA molecules.

In the context of the present invention, it has surprisingly been found that RNA molecules of variable length, including small regulatory RNA molecules, can be detected by a method comprising the use of a degradable 3'-non-extendable oligonucleotide (hereafter also referred to as the "helper oligo"). The method of the present invention is particularly suitable to detect more than one species of RNA molecule in one sample, in particular when the RNA molecules are derived from one precursor molecule. Therefore, the method of the present invention is particularly suitable to detect and discriminate between mature and precursor forms of small regulatory RNAs, including, e.g., the detection and quantification of mature and precursor forms of miRNAs.

Accordingly, in a first aspect, the present invention provides a method for the detection of an RNA molecule, said method comprising the steps of
a) providing a sample containing the RNA molecule;
b) hybridizing to said RNA molecule a first polynucleotide comprising a first primer binding site;
c) extending the first polynucleotide by reverse transcribing the sequence of the RNA molecule to generate a first strand cDNA;
d) hybridizing a second polynucleotide to the first strand cDNA, characterized in that said second polynucleotide is a 3'-non-extendable oligonucleotide comprising
   (i) a 3'-portion complementary to a portion of the first strand cDNA, and
   (ii) a 5'-overhang comprising a sequence of a second primer binding site;
e) extending the first strand cDNA to generate an extension reaction product comprising a sequence complementary to the second primer binding site;
f) amplifying the extension reaction product by means of polymerase chain reaction in the presence of a detection moiety using a first primer complementary to the first primer binding site and a second primer complementary to the second primer binding site; and
g) detecting the amplification product by means of real-time fluorescence readout.

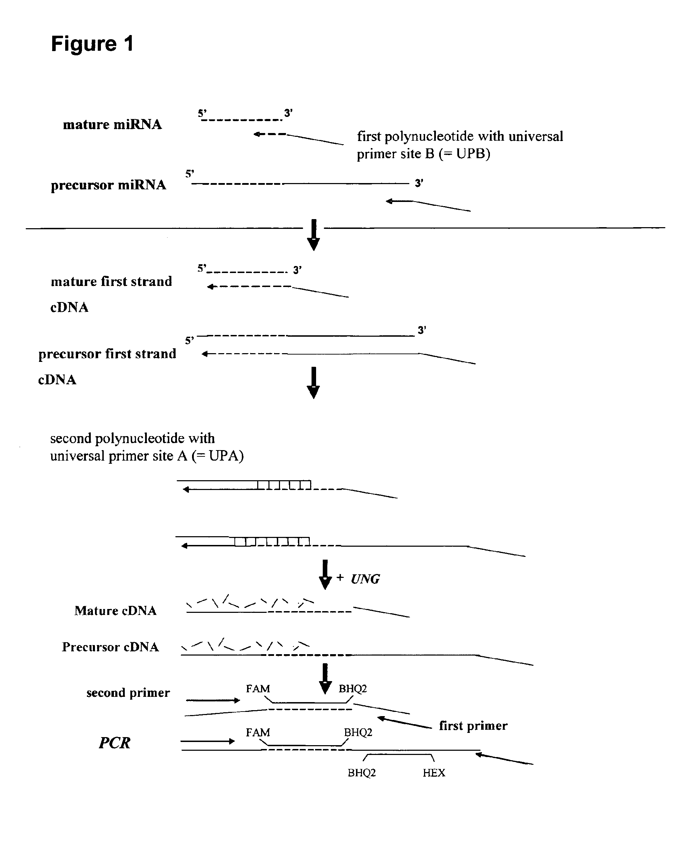

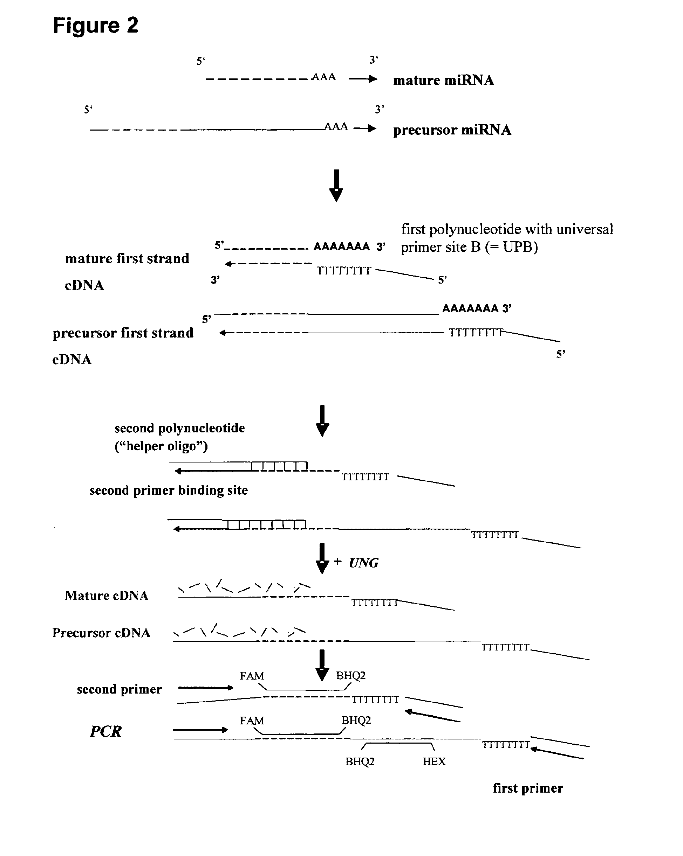

The principles of the method according to the present invention are illustrated in Figures 1 and 2, respectively. In detail, in a first reaction step, a first polynucleotide (also designated herein as the RT-(reverse transcription)-primer) is hybridized to the RNA molecule to be detected. This first polynucleotide is designed to be complementary to a portion of the RNA molecule to be detected, and to further comprise a first primer binding site, i.e. a sequence to which a first primer can specifically bind to. Upon annealing of the first polynucleotide to the RNA molecule, the sequence of the target RNA molecule is reverse transcribed in 3'- to 5' direction by extending the first polynucleotide via the enzymatic reaction of a reverse transcriptase. Thereby, a cDNA molecule complementary to the sequence of the RNA molecule to be detected is generated. Moreover, the length of this cDNA molecule corresponds to the length of the RNA molecule to be detected. In a subsequent reaction step, a second polynucleotide is hybridized to the cDNA which has before been generated by reverse transcription. This second polynucleotide is designed to be (i) complementary to a portion of the cDNA, (ii) to further comprise a 5'-overhang with a second primer binding site, i.e. a sequence to which a second primer can specifically bind to, and (iii) to be 3'-non-extendable. After annealing of the second polynucleotide to its target sequence, the cDNA is further extended in 5'- to 3' direction by reverse transcribing the sequence of the second polynucleotide, thereby generating a DNA extension reaction product with a 5'-portion comprising a sequence complementary to the second primer binding site. By designing the second polynucleotide to be 3'-non-extendable, elongation of the second polynucleotide is prevented during the second reaction step. Highly preferable, if the second polynucleotide is designed to be a degradable polynucleotide, it may then be degraded, e.g. by enzymatic cleavage. The amplification of the extension reaction product is then carried out by polymerase chain reaction in the presence of a first and a second primer which are complementary to the first and second primer binding site, respectively, as well as in the presence of a detection moiety. In case the method of the present invention is applied for the detection of two different RNA molecules in parallel, two extension reaction products can be amplified in one reaction setup when two distinguishable detection moieties are used. Moreover, if the RNA molecules to be detected are of different sizes, different extension reaction products with different lengths may be detected.

The feature "providing a sample" as used in the context of the present invention generally refers to all kind of procedures suitable to prepare a composition containing an RNA molecule or a population of RNA molecules. These procedures include, but are not limited to, standard biochemical and/or cell biological procedures suitable for the preparation of a cell or tissue extract, the cells and/or tissues of which may be derived from any kind of organism containing an RNA molecule of interest. For example, a sample according to the present invention may be purified total RNA and/or size fractionated total RNA derived from a cell or cells grown in cell culture or obtained from an organism by dissection and/or surgery. In particular, a sample according to the present invention may be total RNA obtained from one or more tissue(s) of one or more patient(s). The preparation of total RNA from a cell, from a cell extract or from a tissue may include one or more biochemical purification step such as, e.g., centrifugation and/or fractionation, cell lysis by means of mechanical or chemical disruption steps including, for example, multiple freezing and/or thawing cycles, salt treatment(s) and/or phenol-chloroform extraction. Optionally, providing a sample according to the present invention may also include the removal of large RNA, such as abundant ribosomal rRNA, by precipitation in the presence of polyethylene glycol and salt, and/or methods of denaturing polyacrylamide gel electrophoresis. Methods of purifying total RNA from a cell and/or a tissue are well known to a person skilled in the art and include, e.g., standard procedures such as the use of guanidinium thiocyanate ― acidic phenol-chloroform extraction (e.g. TRIzol®, Invitrogen, USA). Moreover, a sample according to the present invention may further comprise or be of composed of one or more synthetic RNA molecule(s) which may serve as internal standards for quantification. Examples of a sample according to the present invention include, e.g., blood, lung, liver, or any other tissue and/or biopsy sample obtained from an individual.

The term "polynucleotide" as used herein generally refers to a nucleotide molecule of variable length and sequence which is capable of binding to an RNA or DNA target molecule via complementary base pairing. A polynucleotide according to the present invention generally refers to a molecule comprising at least 10, preferably at least 20, and more preferably at least 30 nucleotides. It will be evident to the skilled person that the polynucleotide of the invention has an appropriate length to provide the required specificity. In general, a polynucleotide may be a DNA oligonucleotide or an RNA oligonucleotide, preferably a DNA oligonucleotide. Accordingly, a polynucleotide includes all kind of structures composed of a nucleobase (i.e. a nitrogenous base), a five-carbon sugar which may be either a ribose, a 2'-deoxyribose, or any derivative thereof, and a phosphate group. The nucleobase and the sugar constitute a unit referred to as a nucleoside. The phosphate groups may form bonds with the 2, 3, or the 5-carbon, in particular with the 3 and 5 carbon of the sugar. A ribonucleotide contains a ribose as a sugar moiety, while a deoxyribonucleotide contains a deoxyribose as a sugar moiety. Nucleotides can contain either a purine or a pyrimidine base. The polynucleotide of the invention may further comprise one or more modified nucleotide(s) and/or one or more backbone modification(s) such as, e.g., 2'-O-methyl (2'-OMe) RNA, 2'-fluoro (2'-F), peptide nucleic acids (PNA), or locked nucleic acids (LNA).

The term "primer" as used herein generally refers to an oligomeric compound, or alternatively, to a polynucleotide that is capable to "prime" DNA synthesis by a template-dependent DNA polymerase. That is, the 3'-end of a primer generally encompasses a free 3'-OH group at which further nucleotides may be attached to via the formation of 3' to 5'-phosphodiester linkage. A primer of the present invention may be at least about 10, preferably at least about 20, and more preferably about 30 nucleotides in length. A primer as used in the context of the present invention is designed as such that it specifically anneals and/or binds to its designated primer binding site via base pair complementarity. The term "primer" equally means "polynucleotide" in the context of the present invention. Primers of the present invention are, e.g., exemplified in Table 1 and Table 2 of Example 1 and 2, respectively.

A "primer binding site" as referred to herein means a stretch of nucleotides which is designed as to enable the annealing and/or hybridisation of a complementary a primer or polynucleotide. In the context of the present invention, a primer binding site may be composed of about 10 to 20 nucleotides or more, and may reveal any sequence which is considered appropriate for establishing complementary base pairing to a primer molecule of interest. Moreover, the primer binding site may form part of a longer polynucleotide, and may thus be located near the 5'- end, the 3'- end or at any position in between with respect to the sequence of this polynucleotide. In particular, in the context of the present invention, the first primer binding site forms part of the first polynucleotide and the second primer binding site forms part of a second polynucleotide. This first and second primer binding sites can be either identical to each other, or different from each other. Preferably, the first and second primer binding sites reveal different sequence specificity. Moreover, the primer binding site does preferably not refer to the sequence of the RNA molecule to be detected. Sequences of universal primer binding sites such as, e.g., the T7 or T3 minimal primer sites, are well known to the person skilled in the art, and can, e.g., be obtained from public databases including the NCBI gene bank (National Center for Biotechnology Information, Maryland, USA).

In step b) of the method according to the present invention, a first polynucleotide comprising a first primer binding site is hybridized to the RNA molecule to be detected. This first polynucleotide may either be complementary to the endogenous sequence of the target RNA molecule, or may, alternatively, be complementary to a polynucleotide tail by which the RNA molecule has been extended at its 3'-end. This polynucleotide tail may be any stretch of nucleotides, and is preferably a poly-A tail, i.e. a stretch of multiple adenosine residues.

Accordingly, in one embodiment of the present invention, the first polynucleotide is complementary to the endogenous sequence of the RNA molecule to be detected. In an alternative embodiment, the first polynucleotide is complementary to a particular sequence which has been attached to the 3'-end of the RNA molecule before carrying out step b). When using a first polynucleotide complementary to the sequence of the RNA molecule, i.e. a sequence-specific first polynucleotide (principle of method I, see Fig. 1), those nucleotides of the first polynucleotide which are complementary to the sequence of the RNA molecule should preferably not overlap with the sequence of the second polynucleotide used in step c). Moreover, the specificity of the first polynucleotide can be increased by the parallel use of a dU-DNA oligonucleotide that blocks the sequence of the first primer binding site during the process of reverse transcription, i.e. during step c). When using a first polynucleotide complementary to a sequence which has been attached to the 3'-end of the RNA molecule (principle of method II, see Fig. 2), the use of anchored polynucleotides is preferred. In the context of the present invention, anchored polynucleotides mean polynucleotides that carry at least one or more nucleotides specific for the target RNA molecule prior to the attached polynucleotide tail sequence. In this setup, tailing of the RNA molecule before reverse transcription is required, particularly if the RNA is not a poly-adenylated mRNA.

The term "complementary to" as referred to in the context of the present invention generally means the capability of a polynucleotide to specifically bind to a target sequence of interest by means of complementary base pairing. Complementary base pairs are formed between two nucleotide molecules (which may optionally include modifications) that are complementary to each other. In the context of the present invention, complementary base pairs which are, e.g., formed between the first polynucleotide and the RNA target molecule, may include all kind of canonical or non-canonical base pairs, including, but not limited to, Watson-Crick A-U, Watson-Crick A-T, Watson-Crick G-C, G-U Wobble base pairs, A-U and A-C reverse Hoogsteen base pairs, or purine-purine and pyrimidine-pyrimidine base pairs such as sheared G-A base pairs or G-A imino base pairs. Preferably, complementary base pairs refer to canonical base pairs in the context of the present invention.

The term "hybridizing" or "hybridization" as used herein generally means the annealing of two complementary strands. Successful hybridization depends on a variety of factors, including temperature, salt concentrations, and/or pH. The optimal temperature for hybridization is preferably in the range of 5-15 °C below the Tₘ value which defines the melting temperature (Tₘ) of hybrids, i.e. the temperature at which 50 % of the double-stranded nucleic acid chains are separated. Various formulas for calculating Tₘ values are known to the person in the art. Conditions conducive for hybridizing in the context of the present invention may include the use of buffer containing reagents to maximize the formation of duplex and to inhibit non-specific binding of the polynucleotide to its target molecule. If required, the final concentration of the polynucleotide may be optimized for each reaction. Conditions conducive for hybridization also include incubating the polynucleotide with the target molecule for a sufficient period of time to allow optimal annealing. Preferably, hybridizing according to the present invention refer to hybridization conditions in which the polynucleotide is incubated with a target molecule in solution. Hybridization conditions of the present invention are, e.g., illustrated in method I and method II of Example 1 and 2. Moreover, it is advantageous to optimize temperature conditions during polymerase reactions such as first strand cDNA synthesis and elongation subsequent to the hybridization of the helper oligonucleotide. Such an optimization is easily to perform by the person skilled in the art.

The term "3'-non-extendable oligonucleotide" as used generally herein means a polynucleotide composed of ribonucleotides, deoxynucleotides or both, but without a reactive, i.e. a free, 3'-OH group at the 3'-terminal end. In the absence of a reactive 3'-OH group, extension and/or elongation of the oligonucleotide in 5'- to 3'-direction by the enzymatic addition of further nucleotides is prevented. Preferably, a 3'-non-extendable oligonucleotide according to the present invention contains a phosphate group at its 3'-end. In the context of the present invention, the terms "polynucleotide" and "oligonucleotide" are equally used. Moreover, a 3'-non-extendable oligonucleotide of the invention is characterized in that it comprises a 3'-portion with sequence complementarity to its target sequence, while having a 5'-portion comprising a sequence of a second primer binding site. This 5'-portion, however, does not anneal to the target sequence, thereby generating a 5'-overhang. In the context of the present invention, the term "5'-overhang" is well known to a person skilled in the art, and further illustrated in Figures 1 and 2.

As used herein, the term "amplifying" or "amplification" refers to the process of synthesizing nucleic acid molecules that are complementary to one or both strands of a template nucleic acid (e.g., the first strand cDNA and/or the extension reaction product as generated in step e) of the method according to the present invention). Amplifying a nucleic acid molecule typically includes denaturing the template nucleic acid, annealing primers to the template nucleic acid at a temperature that is below the melting temperatures of the primers, and enzymatically elongating from the primers to generate an amplification product. The denaturing, annealing and elongating steps each can be performed once. Generally, however, the denaturing, annealing and elongating steps are performed multiple times such that the amount of amplification product is increasing, oftentimes exponentially, although exponential amplification is not required by the present methods. Amplification typically requires the presence of deoxyribonucleoside triphosphates, a DNA polymerase enzyme (e.g. Taq Polymerase) and an appropriate buffer and/or cofactors for optimal activity of the polymerase enzyme (e.g., MgCl₂ and/or KCl). Amplification conditions of the present invention are, e.g., described in Example 1, Section 6 and 7.

The term "polymerase chain reaction" or "PCR" as used in the context of the present invention generally means any assay or procedure by which a template molecule is specifically amplified. Polymerase chain reaction is a standard method known to the person skilled in art in which a template molecule is a nucleic acid template. In the context of the present invention, the template molecule may be a DNA or RNA molecule, preferably a cDNA molecule generated from an RNA molecule by primer extension and reverse transcription. The template nucleic acid does not necessarily need to be purified; it may be present in only minor amounts such as, for example, RNA molecules which are only expressed in low abundance. In order to amplify the extension reaction product by means of polymerase chain reaction, the primers are combined with other PCR reagents under reaction conditions that induce primer extension. For example, chain extension reactions generally include 50 mM KCI, 10 mM Tris-HCl (pH 8.3), 1.5 mM MgCl₂, up to 1.0 µg (e.g. 0.5 to 1.0) denatured template DNA, 50 pmoles of each oligonucleotide primer, 2.5 U of Taq polymerase, and 10 % DMSO. The reactions usually contain 150 to 320 µM of dATP, dCTP, dTTP, dGTP, or one or more analogs thereof. In certain circumstances, 300 to 640 µM dUTP can be substituted for dTTP in the reaction. In the context of the present invention, polymerase chain reaction is carried out in the presence of a first and a second primer as well as in the presence of a detection moiety. The experimental conditions of the polymerase chain reaction according to the present invention are, e.g., described in Example 1.

During the process of polymerase chain reaction, the newly synthesized strands form a double-stranded molecule that can be used in the succeeding steps of the reaction. The steps of strand separation, annealing, and elongation can be repeated as often as needed to produce the desired quantity amplification products corresponding to the target nucleic acid molecule. The limiting factors in the reaction are the amounts of primers, thermostable enzyme, and nucleoside triphosphates present in the reaction. The amplification step and the hybridization step are preferably repeated at least once. For use in detection, the number of amplification and hybridization steps will depend, e.g., on the nature of the sample. If the sample comprises only a few copies of target nucleic acids, more amplification and hybridization steps may be required to amplify the target sequence sufficient for detection. Generally, the amplification and hybridization steps are repeated at least about 15 times, but may be repeated as many as at least 20, 30, or even 40 times. A thermostable polymerase refers to a polymerase enzyme that is heat stable, i.e., which does not irreversibly denature when subjected to the elevated temperatures for the time necessary to effect denaturation of double-stranded template nucleic acids. Generally, the synthesis is initiated at the 3' end of each primer and proceeds in the 5' to 3' direction along the template strand. Thermostable polymerases have been isolated from *Thermus flavus, T. ruber, T. thermophilus, T. aquaticus, T. lacteus, T. rubens, Bacillus stearothermophilus,* and *Methanothermus fervidus.* Nonetheless, polymerases that are not thermostable also can be employed in PCR provided the enzyme is replenished. Optionally, in the context of the present invention, the polymerase chain reaction may be carried out in the presence of the enzyme Uracil-DNA glycosylase (UNG).

In the context of the present invention, the terms "detection" or "detecting" generally mean visualizing, analyzing and/or quantifying the amplification product of the present invention. In particular, the term "detecting" refers to any method known in the art which is applicable to detect the amplification product by means of fluorescence readout, preferably by means of real-time RT-PCR.

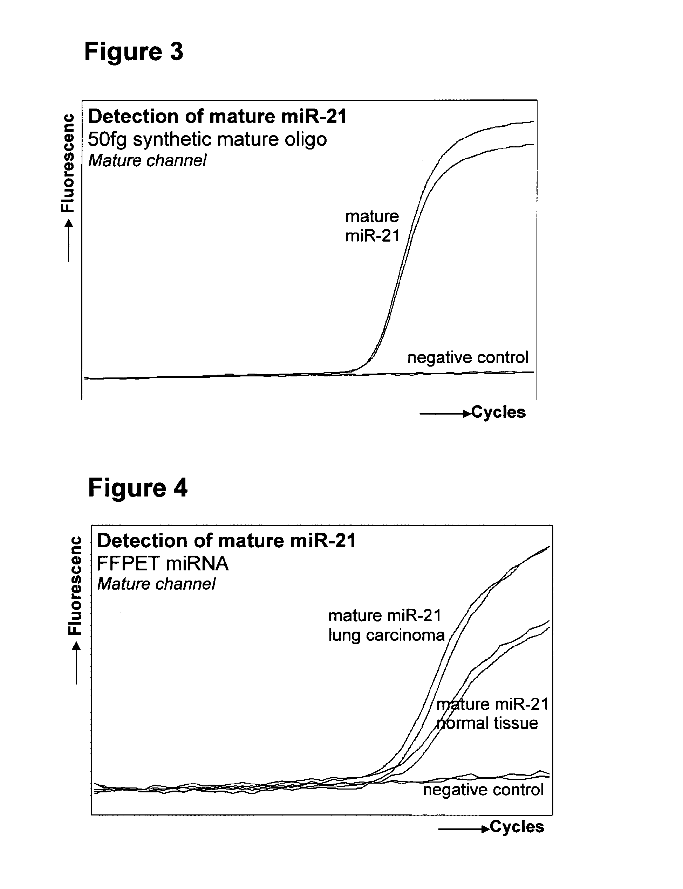

The term "real-time fluorescence readout" generally means all kind of imaging methods known in the art that are suitable to visualize, detect, analyze and/or quantify the amplification product of the present invention in real time, i.e. while the process of amplification. In particular, real-time fluorescence readout refers to a real-time polymerase chain reaction (RT-PCR, also called quantitative real time polymerase chain reaction, i.e. Q-PCR/qPCR) which is a method based on the polymerase chain reaction and which is suitable for amplifying and simultaneously quantifying a target DNA molecule. RT-PCR enables both detection and quantification (as absolute number of copies or relative amount when normalized to DNA input or additional normalizing genes) of one or more specific sequences in a DNA sample. In real-time PCR or RT-PCR, fluorescence is measured during each cycle of PCR reaction, while the amount of fluorescence is proportional to the amount of PCR product. In general, the two common methods for detection of products in real-time PCR are: (1) non-specific fluorescent dyes that intercalate with any double-stranded DNA, and (2) sequence-specific DNA probes labelled with a fluorescent reporter moiety which permits detection only after hybridization of the probe with its complementary DNA target. Preferably, real-time fluorescence readout according to the present invention includes, but is not limited to, the real-time imaging of the amplification product using a Lightcycler® system (Roche, Germany). The principles of real-time PCR and/or RT-PCR are well known to a person skilled in the art and are, e.g., described in "Critical Factors for Successful Real-Time PCR", Qiagen, Germany. Results obtained by real-time fluorescence readout in the context of the present invention and further exemplified in Figures 3 to 5.

The term "detection moiety" as used herein generally refers to any substance or agent which can be incorporated into a DNA or, alternatively, which can be attached and/or linked to a polynucleotide, and which can be employed to visualize and/or quantitate the amplification product of interest by fluorescent measurement. A detection moiety according to the present invention may be an intercalating dye such as, e.g., SyberGreen®, or a non-intercalating moiety such as a fluorophore. SyberGreen® binds to all double-stranded DNA molecules and emits a fluorescent signal of a defined wavelength upon binding. The use of intercalating dyes enables the analysis of many different targets without the need of synthezising target-specific labeled probes. However, since non-specific RNA products and primer-dimer will also contribute to the fluorescence signal, high PCR specificity is required when using intercalating dyes such as SyberGreen®. Fluorophores include, but are not limited to, fluorescein dyes, rhodamine dyes, or cyanine dyes. Fluorescent labels are commercially available from diverse suppliers including, for example, Invitrogen™ (USA).

The method of the present invention is applicable for the detection of RNA molecules of variable length. In the context of the present invention, however, it has been found that the method as described herein is particularly suitable for the detection of small RNA molecules.

Accordingly, in a preferred embodiment, the RNA molecule has a length of from 15 to 200 nucleotides, preferably of from 20 to 100 nucleotides.

That is, in the context of the present invention, the RNA molecule to be detected may have a length of from 10 to 250 nucleotides, or from 15 to 200. nucleotides, or from 40 to 120 nucleotides, or preferably a length of from 20 to 100 nucleotides. However, it is evident to the skilled person that the above upper and lower limits may also be combined in order to arrive at different ranges. Accordingly, the RNA molecule may also have a length of from 15 to 120, or from 40 to 100, of from 80 to 250. Moreover, the sample of the invention containing may contain a population of RNA molecules with variable lengths. That is, the sample provided in the context of the present invention may comprise RNA molecules of the same length, or may comprise RNA molecules with different length, including, but not limited to, precursor and mature forms of an RNA molecule of interest. Preferably, the sample provided in the context of the present invention comprises RNA molecules of distinguishable lengths, such as, for example, the precursor and the mature form of an RNA molecule. Examples of these include, but are not limited to, precursor miRNA and mature miRNA which may vary about 50 to 100 nucleotides in lengths.

In a further preferred embodiment, the RNA molecule is selected from the group consisting of mature miRNA (miRNA), precursor miRNA (pre-miRNA), primary miRNA precursor (pri-miRNA), small interfering RNA (siRNA), piwi-interacting RNA (piRNA), precursor piRNA, and short hairpin RNA (shRNA).

The terms "mature miRNA", "miRNA" or "microRNA", which are equally used in the context of the present invention, generally refer to an RNA molecule of short length which is expressed within a cell. In particular, the term "miRNA" refers to a single-stranded RNA of about 20 to 25 nucleotides in length which is generated from an endogenous hairpin-shaped precursor molecule of approximately 70 nucleotides in length. Genes encoding miRNAs are found in the genomes of humans, animals, plants and viruses, respectively.

miRNA encoding genes are much longer than the processed mature miRNA molecule. That is, miRNAs are first transcribed as primary transcripts (also designated as "primary miRNA precursors") with a cap and poly-A tail and processed to short, 70-nucleotide stem-loop structures known as "precursor-miRNA" (pre-miRNA) in the cell nucleus. This processing is performed in animals by a protein complex known as the Microprocessor complex, consisting of the nuclease Drosha and the double-stranded RNA binding protein Pasha. These pre-miRNAs are then processed to mature miRNAs in the cytoplasm by interaction with the endonuclease Dicer, which also initiates the formation of the RNA-induced silencing complex (RISC). This complex is responsible for the gene silencing observed due to miRNA expression and RNA interference.

The term "small-interfering RNA" or "siRNAs" generally means an RNA molecule which is produced upon exogenous delivery of a dsRNA molecule into a cell, upon transgenic expression of long dsRNA, or which is introduced into a cell by gene transfer, cell transfection or cell transduction, or which is endogenously expressed in a cell. The term "siRNA" also means a short regulatory RNA molecule which is implicated in RNA interference and gene silencing, preferably resulting in the degradation of a target RNA transcript. A small-interfering RNA may be a single-stranded RNA or may be a double-stranded RNA consisting of two separate RNA strands, i.e. a sense and an antisense strand. Small-interfering RNAs are generally 20-25 nucleotides in length.

The term "piwi-interacting RNA" or "piRNA" generally refers to an RNA molecule which is functionally linked to silencing transposons and maintaining genome integrity during germline development, in particular during spermatogenesis. Piwi-interacting RNAs form part of RNA-protein complexes through interactions with Piwi proteins and are distinct from miRNAs in size in that they preferably have a length of 26―31 nucleotides. The term "piRNA" as used herein also includes the subclass of regulatory small RNAs called "Repeat associated small interfering RNA" or "rasiRNA". RasiRNA associate with both the Ago and Piwi Argonaute protein subfamily while piRNA only associates with the Piwi Argonaute subfamily. In the germline, rasiRNA is involved in establishing and maintaining heterochromatin structure, controlling transcripts that emerge from repeat sequences, and silencing transposons and retrotransposons. RasiRNA is also distinct in its size. Contrary to miRNAs which are 21-23 nucleotides in length, siRNAs which are 20-25 nucleotides in length, and piRNAs which are 24-31 nucleotides in length, rasiRNAs are 24-29 nucleotides in length depending on the organism derived from. The different subclasses of miRNA, siRNA and piRNA can also be distinguished by their way of biogenesis in that miRNA require the enzyme Dicer-1 for its production, siRNA requires the enzyme Dicer-2, while rasiRNA does not require either.

The term "short hairpin RNA" or "shRNA" generally refers to a short RNA molecule with a stem-loop hairpin structure. A short hairpin RNA may also be a stem-loop RNA structure derived from an endogenous template, including, but not limited to, a gene encoding vector or plasmid. In particular, a short hairpin RNA means an RNA molecule which makes a tight hairpin turn and can be used to silence gene expression.

In a preferred embodiment of the first aspect of the present invention, the RNA molecule of step a) is extended at the 3'-terminus by a polynucleotide tail, preferably via enzymatic reaction of a poly(A) polymerase, a terminal transferase, or a ligase, more preferably via enzymatic reaction of a poly(A) polymerase.

The term "polynucleotide tail" as used herein generally designates a stretch of nucleotides at the 3'- end of an RNA molecule. A polynucleotide tail according to the present invention may be of variable length, and preferably comprises about 20 to 200 nucleotides. The polynucleotide tail according to the present invention may be composed of any kind of nucleotides, and may also include any kind of modified nucleotides if considered appropriate. In the context of the present invention, a polynucleotide tail is designed as such that a primer or a polynucleotide can be hybridized thereto under suitable conditions.

If generated by the enzymatic reaction of a poly(A) polymerase, the polynucleotide tail of the invention is preferably composed of adenosine residues.

In the context of the present invention, a "poly(A) polymerase" generally means an enzyme which catalyzes the addition of adenosine to the 3'-end of an RNA molecule in a sequence-independent fashion. In particular, a poly(A) polymerase of the present invention means a polynucleotide adenylyltransferase having the enzyme classification of EC 2.7.7.19, or alike.

The term "terminal transferase" generally refers to an enzyme that catalyzes the addition of nucleotides to the 3'- terminus of a DNA molecule, and preferably, as in the context of the present invention, to the 3'- terminus of an RNA molecule. The terminal transferase of the invention preferably adds nucleotides to protruding 3' ends, but will also, less efficiently, add nucleotides to blunt and 3'- recessed ends of DNA fragments.
The term "ligase" as used herein means an enzyme that can catalyse the joining of two large molecules by forming a new chemical bond by catalyzing the formation of a phosphodiester at the site of a single- strand break in duplex DNA. A ligase of the present invention is preferably classified as EC 6.5.1.1 and can also act on RNA substrates.

In another preferred embodiment, the method of the present invention is characterized in that first polynucleotide is complementary to the sequence of the RNA molecule, complementary to the polynucleotide tail, or complementary to both.

As detailed above, the first polynucleotide which is hybridized to the RNA molecule in step b) of the present method may be either a sequence-specific polynucleotide, which is complementary to the target nucleic acid or a polynucleotide which is complementary to the polynucleotide tail, which is a poly-A tail, when poly-A polymerase has been used for 3' extension. When using a polynucleotide with sequence complementarity to the polynucleotide tail, the use of an oligo-dT polynucleotide is preferred. An oligo-dT polynucleotide means an polynucleotide composes of desoxythymidine residues.

In the context of the present invention, it has further surprisingly been found that degradable polynucleotides (also referred to as "helper oligos") can be used for any application where addition of and/or elongation by a certain sequence to one or both strains of a DNA molecule is required. The use of degradable polynucleotides is an alternative to ligation reactions or to the use of PCR primer with tails, and has the advantage that the exact original sequence to which the degradable polynucleotide hybridizes will be amplified and detected.

Accordingy, in yet another preferred embodiment, the method of the present invention is characterized in that the second polynucleotide is a degradable polynucleotide.

The term "degradable polynucleotide" as used generally refers to any polynucleotide which can be degraded by enzymatic, chemical or physical treatment. In particular, a degradable polynucleotide of the present invention means a polynucleotide which is designed as such that it can be specifically digested, for example, by the enzyme Uracil-DNA-glycosylase (UNG). The enzyme Uracil-DNA-glycosylase (UNG) catalyzes the hydrolysis of the N-glycosylic bond between uracil and sugar, leaving an apyrimidinic site in uracil-containing single or double-stranded DNA. Therefore, in order to be digestible by the enzyme Uracil-DNA-glycosylase (UNG), the degradable polynucleotide of the inventions should comprise desoxyuracil (dU) nucleotide residues. The incorporation of dU-nucleotide residues into a polynucleotide including their enzymatic digestion in the presence of Uracil-DNA-glycosylase is common knowledge to the person skilled in the art, and, e.g., described in U.S. Patent Nos. 5,035,996, 5,683,896 and 5,945,313.

Accordingly, in a particularly preferred embodiment, the second polynucleotide comprises dU-nucleotide residues and is digested after step e) by the enzymatic reaction of Uracil-DNA-glycosylase (UNG).

In particular, degradation of the polynucleotide (i.e. the "helper oligo") by treatment with heat labile Uracil-DNA-glycosylase (UNG) can be part of the PCR reaction (e.g. when UNG is included in the PCR enzyme mix). The used UNG has to be heat labile if the following PCR does incorporate dUTP.

In an alternative embodiment, the degradable polynucleotide may also be an RNA polynucleotide. In this case, the first denaturation step during the PCR reaction will degrade the RNA polynucleotide if the buffer contains the usual PCR buffer salt concentrations.

Preferably, the 5' part of the degradable polynucleotide comprises a sequence of a second primer binding site, and the 3' part comprises the specific sequence of the 5' part of the target RNA molecule. For example, this sequence-specific part of the degradable polynucleotide can be designed to fit on only mature or on only precursor or on both forms of the target RNA molecule. Alternatively, the degradable polynucleotide of the invention may also be composed of RNA nucleotides.

In a further preferred embodiment, the method of the present invention is characterized in that the second polynucleotide has a blocked 3'-terminus in form of a 3'-terminal phosphate.

As detailed above, a 3'-terminal phosphate group efficiently blocks any enzymatic elongation of the polynucleotide, i.e. the attachment of additional nucleotides during the course of the polymerase chain reaction. A blocked 3'-terminus of the second polynucleotide may also be obtained by any other suitable modification known to the person skilled in the art. The embodiment of a blocked 3'-terminus is of particular advantage in the context of the present method.

In a preferred embodiment, the method of the present invention is characterized in that the detection moiety in step f) is an intercalating dye.

The term "intercalating dye" as used herein generally means any dye which binds to double-stranded DNA. In particular, an intercalating dye of the present invention is a dye that binds to double-stranded DNA but not to single-stranded DNA and which fluorescence increases once it is bound to the double-stranded DNA, examples of which include, e.g., commercially available dyes such as SybrGreen® (Invitrogen, USA). An intercalating dye according to the present invention binds to all double-stranded DNA in PCR, causing the fluorescence of the dye. An increase in the DNA product during PCR therefore leads to an increase in fluorescence intensity and is measured at each cycle, thus allowing DNA concentrations to be quantified. However, dsDNA dyes such as SybrGreen® will bind to all dsDNA PCR products, also including non-specific PCR products such as, e.g., "primer dimers". This may potentially interfere with or prevent accurate quantification of the intended target sequence. In general, the PCR reaction is prepared as usual, with the addition of the fluorescent intercalating dye. The reaction is run in a thermocylcer, and after each cycle, the levels of fluorescence are measured with a detector; the dye only fluoresces when bound to the dsDNA (i.e., the PCR product). With reference to a standard dilution, the dsDNA concentration in the PCR can be determined. Like other real-time PCR methods, the values obtained as such do not have absolute units associated with it (i.e. mRNA copies/cell). A comparison of a measured DNA/RNA sample to a standard dilution will only give a fraction or ratio of the sample relative to the standard, allowing only relative comparisons between different tissues or experimental conditions. To ensure accuracy in the quantification, it is usually necessary to normalize the expression of a target gene to a stably expressed gene. This can correct possible differences in RNA quantity or quality across experimental samples.

In another preferred embodiment, the method of the present invention is characterized in that the detection moiety in step f) is a hydrolysis probe.

The term "hydrolysis probe" as used herein generally refers to an oligonucleotide which can be enzymatically hydrolysed. In particular, in the context of the present invention, a "hydrolysis probe" refers to an oligonucleotde which can be hydrolysed by the 5'- to 3' exonuclease activity of a Taq DNA polymerase during a PCR reaction. More preferably, a "hydrolysis probe" of the present invention means a TaqMan® probe, i.e. a sequence-specific oligonucleotide carrying both a fluorophore and a quencher moiety, in which the fluorophore and the quencher moiety are attached to the oligonucleotide as such that the proximity of the fluorophore (i.e. the fluorescent reporter or the fluorescent label) to the quencher prevents the reporter from fluorescing. The use of TaqMan® probes in quantitative, real-time PCR analysis is well known procedure known to the person skilled in the art, and, e.g., exemplified in Example 1. In general, TaqMan® probes are designed as such that the fluorophore is attached at or near the 5'- end of the oligonucleotide probe, while the quencher is located at or near the 3'- end. During the combined annealing/extension phase of a polynucleotide chain reaction (PCR), the probe is cleaved by the 5'-to 3' exonuclease activity of Taq DNA polymerase, thereby separating the fluorophore and the quencher moieties, with the consequence that the fluorescence reporter can emit a fluorescence signal which can then be measured. The detectable fluorescence is proportional to the amount of accumulated PCR product.

In the context of the present invention, a fluorophore (i.e. a fluorescent reporter or a florescent label) attached to a hydrolysis probe may be selected from, but is are not limited to, the group of fluorescein dyes such as carboxyfluorescein (FAM), 6-carboxy-4',5'-dichloro-2'7'-dimethoxyfluorescein (JOE), fluoresceinisothiocyanat (FITC), tetrachlorofluorescein (TET), or 5'-Hexachloro-Fluorescein-CE Phosphoramidite (HEX); rhodamine dyes such as, e.g., carboxy-X-rhodamine (ROX), Texas Red and tetramethylrhodamine (TAMRA), cyanine dyes such as pyrylium cyanine dyes, DY548, Quasar 570, or dyes such as Cy3, Cy5, Alexa 568, or alike. The choice of the fluorescent label is typically determined by its spectral properties and by the availability of equipment for imaging. The use of fluorescent labels in quantitative assays is a standard procedure well known to the person skilled in the art, and fluorophores are commercially available from several suppliers including, e.g., Invitrogen®, USA.

A quencher generally refers to a molecule which absorbs energy transferred from a donor molecule (i.e. a fluorescent reporter). That is, the donor molecule transfers energy to the quencher, and the donor returns to the ground state and generates the excited state of the quencher. Fluorescence quenching also depend on the distance between the donor and the acceptor molecule. Until the last few years, quenchers have typically been fluorescent dyes, for example, fluorescein as the reporter and rhodamine as the quencher (FAM/TAMRA probes). One of the best known quenchers is TAMRA (tetramethylrhodamine) which is used to lower the emission of the reporter dye. Due to its properties TAMRA is suitable as a quencher for FAM (carboxyfluorescein), HEX (hexachlorofluorescein), TET (tetrachloro-fluorescein), JOE (5'-Dichloro-dimethoxyfluorescein) and Cy3-dyes (cyanine). Alternatively, a "quencher" according to the present invention may be a non-fluorescent (so called dark) quencher which generally enables multiplexing. Dark quencher which may be applicable in the context of the present invention include, but are not limited to, agents such as, e.g. DABCYL (4-[[4-(dimethylamino)-phenyl]-azo]-benzoic acid) which quenches dyes in a range of from 380 to 530 nm, the Eclipse^{®} Quencher (4-[[2-chloro-4-nitro-phenyl]-azo]-aniline, trademark of Epoch Biosciences, Inc., Corporation Delaware 21720, 23^{rd} Drive NE, Suite 150, Bothell Washington 98021, USA) which has an absorption maximum at 530 nm and efficiently quenches over a spectrum from 520 to 670 nm, or Black Hole Quenchers, such as BHQ-1 ([(4-(2-nitro-4-methyl-phenyl)-azo)-yl-((2-methoxy-5-methyl-phenyl)-azo)]-aniline) and BHQ-2 ([(4-(1-nitro-phenyl)-azo)-yl-((2,5-dimethoxy-phenyl)-azo)]-aniline) (all available from Biosearch Technologies, Inc.) which are capable of quenching across the entire visible spectrum. These non-fluorescent acceptors are often applied as alternative to fluorescent acceptors in order to decrease background fluorescence and in this way sensitivity.

In the context of the present invention, the hydrolysis probe is utilized to detect the presence or absence of an amplification product. As detailed above, this technology utilizes single-stranded hybridization probes labelled with one fluorescent moiety and one quenching moiety. During the annealing step of the PCR (polymerase chain reaction), the labelled hydrolyzation probe binds to the target DNA (i.e., the amplification product) and is degraded by the 5' to 3' exonuclease activity of the Taq Polymerase during the subsequent elongation phase. As a result, the excited fluorescent moiety and the quenching moiety become spatially separated from each other. As a consequence, upon excitation of the fluorophore when no quencher is in close proximity, the fluorescence emission can be detected. By way of example, an ABI^{®} PRISM 7700 Sequence Detection System (trademark of Applied Biotechnology Institute, Inc. Corporation Iowa, Building 36, Cal Poly State University San Luis Obispo, California 93407, USA) uses hydrolysis probe technology. Information on PCR amplification and detection using an ABI^{®} PRISM 7700 system can be found at http://www.appliedbiosystems.com/products.

Hydrolysis probes according to the present invention as well as their use in the method of the present invention are, e.g., described in Examples 1 and 2, respectively.

The method of the present invention has further successfully been applied to detect more than one RNA molecule in one reaction set up. That is, in a preferred embodiment, the method of the invention is applied as such that at least two different RNA molecules are detected in parallel. In this setup, the RNA molecules can be either of different length or of different identity, or of both. As a prerequisite for the parallel detection of at least two RNA molecules in one reaction, however, amplification of the extension reaction products should be carried out in the presence of a detection moiety, wherein the detection moiety is not an intercalating dye. Preferably, in order to detect at least two different RNA molecules in one reaction, amplification of the corresponding extension reaction products in step f) should be carried out in the presence of sequence-specific hydrolysis probes.

Hence, in a preferred embodiment, the method of the present invention is characterized in that at least two different extension reaction products are amplified in step f).

As detailed above, the term "extension reaction product" refers to the molecule generated in step e) of the present method. That is, the "extension reaction product" is a molecule corresponding to the sequence of the RNA molecule which serves as a template for the amplification in step f).

More preferably, the method of the present invention is characterized in that the amplification in step f) is carried out in the presence of at least two hydrolysis probes, wherein at least one of said hydrolysis probes is specific for a specific species of RNA molecules, and wherein the probes comprise different sets of donor/acceptor moieties.

As detailed above, each hydrolysis probe is labeled with a fluorophore which serves as a fluorescent reporter or as a fluorescent label. In the context of the present invention, said fluorophore/fluorescent reporter/fluorescent label is thus to be understood as a "donor moiety". Moreover, each hydrolysis probe is additionally labeled with a quencher. This quencher, which can also be a fluorophore, is generally to be understood as an "acceptor moiety" in the context of the present invention.

Suitable donor and acceptor moieties are known in the art and the skilled practitioner is able to choose a suitable combination of a donor and a corresponding acceptor molecule. As used herein with respect to donor and corresponding acceptor fluorescent moieties, "corresponding" refers to an acceptor fluorescent moiety having an emission spectrum that overlaps the excitation spectrum of the donor fluorescent moiety. However, both signals should be separable from each other. Accordingly, the wavelength maximum of the emission spectrum of the acceptor fluorescent moiety preferably should be at least 30 nm, more preferably at least 50 nm such as at least 80 nm, at least 100 nm or at least 140 nm greater than the wavelength maximum of the excitation spectrum of the donor fluorescent moiety.

Preferred donor fluorescent moieties according to the present invention are fluorescent labels, which include, but are not limited to, e.g., fluorescent dyes such as fluorescein dyes, rhodamine dyes, cyanine dyes, and coumarin dyes. For example, the donor fluorescent moiety may be fluorescein, while the acceptor fluorescent moiety may be selected from the group consisting of LC-Red 610, LC-Red 640, LC-Red 670, LC-Red 705, Cy5, and Cy5.5, preferably LC-Red 610 or LC-Red 640. More preferably, the donor fluorescent moiety is fluorescein and the acceptor fluorescent moiety is LC-Red 640 or LC-Red 610. The donor and acceptor fluorescent moieties can be attached to the appropriate hydrolysis probe via a linker. The length of each linker can be important, as the linker will affect the distance between the donor and the acceptor fluorescent moieties. The length of a linker arm for the purpose of the present invention is the distance in Angstroms from the nucleotide base to the fluorescent moiety.

Alternatively, the acceptor moiety may be a quencher, preferably a dark quencher.

In the context of the present invention, the combination of carboxyfluorescein (FAM) as a donor fluorescent moiety and BHQ-2 ([(4-(1-nitro-phenyl)-azo)-yl-((2,5-dimethoxyphenyl)-azo)]-aniline) as an acceptor moiety has been found particularly suitable for use in the present method. The same applies for the combination of 5'-Hexachloro-Fluorescein-CE-Phosphoramidite (HEX) as a donor fluorescent moiety and BHQ-2 ([(4-(1-nitrophenyl)-azo)-yl-((2,5-dimethoxy-phenyl)-azo)]-aniline) as an acceptor moiety (i.e. dark quencher).

Accordingly, preferred combinations of donor/acceptor moieties are carboxyfluorescein (FAM) and BHQ-2 ([(4-(1-nitro-phenyl)-azo)-yl-((2,5-dimethoxy-phenyl)-azo)]-aniline), and 5'-Hexachloro-Fluorescein-CE Phosphoramidite (HEX) and BHQ-2 ([(4-(1-nitrophenyl)-azo)-yl-((2,5-dimethoxy-phenyl)-azo)]-aniline).

In the context of the present invention, it has further be found that RNA molecules of different lengths which are derived form the same species can be detected in one reaction when using hydrolysis probes labeled with different donor/acceptor moieties.

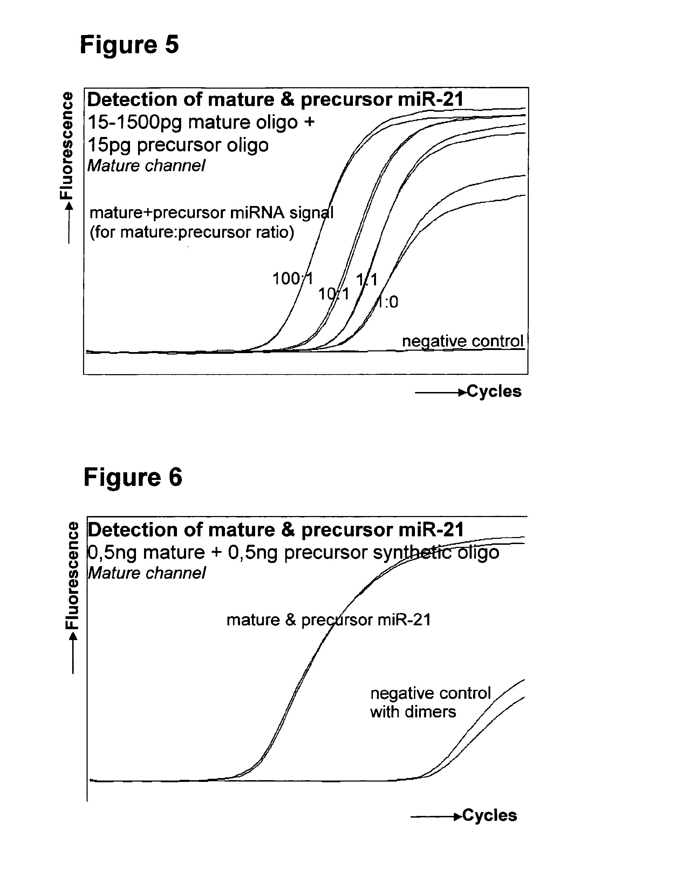

That is, the method of the present invention can be applied to detect two different species of RNA molecules, wherein the two different species of RNA molecules are derived from one precursor molecule. In that case, the method of the invention can be applied to detect and amplify both species of RNA molecules in one separate reaction by dual color detection and quantification. Examples of this scenario include, but are not limited to, the detection of mature miRNA (miRNA) versus precursor miRNA (pre-miRNA). In particular, here, the design of the polynucleotids and/or primer results in the detection of mature plus precursor signal in the mature channel, since the mature probe also binds to the mature sequence in the precursor. The precursor channel detects the precursor signal only. It is apparent for a person skilled in the art that the different design of the first and second polynucleotide will allow for the detection of mature signal only in the mature channel. The first and the second polynucleotide can be the same for the different miRNA forms, i.e. the mature and precursor miRNA. The detection of two RNA molecules having different lengths and derived from one precursor molecule is, e.g., exemplified in Figure 5.

Accordingly, in a preferred embodiment, the specific species of RNA molecules are derived from one precursor molecule, preferably wherein the RNA molecules have different lenghts.

In the context of the present invention, it has been found that the design of the first and second polynucleotide determines whether both forms (e.g. mature and precursor miRNA) or only one form of the RNA species is detected. That is, the precursor form of a target miRNA can also be amplified and detected by an internal PCR primer generating a smaller amplicon (for example of the same size as with the mature form) of this target miRNA. In this case, the hydrolysis probe must be designed to fit between the first and the second polynucleotide. The precursor form will then no longer be detected in both channels, but in the precursor channel only. Accordingly, quantification of mature and precursor is separate. In this case, no elongation of the precursor form by a degradable polynucleotide is required. Instead, for amplification of the precursor miRNA the precursor specific first polynucleotide and a second precursor specific polynucleotide are used.

Accordingly, in another preferred embodiment, the method of the present invention is characterized in that the ratio between the different species of RNA molecules is determined by the detection of different amplification products, preferably by the detection of different amplification products with distinguishable fluorescent readout.

It is evident for a person skilled in the art that the detection of different amplification products will include the use of different hydrolysis probes with different donor/acceptor moieties which may give rise to distinguishable fluorescent signals. Typical reactions for determining the ratio between different species of RNA molecules include the relative quantification of the different amplification products. Relative quantification determines the ratio between the amount of a target RNA molecule and an endogenous reference molecule, which is usually a suitable housekeeping gene. This normalized value can then be used to compare, for example, differential gene expression in different samples.

Alternatively, relative quantification can be performed by applying the comparative ΔΔC_{T} method which relies on direct comparison of C_{T} values. The C_{T} value is the threshold cycle and means the cycle at which the amplification plot crosses the threshold, i.e., at which there is a significant detectable increase in fluorescence. Generally, the C_{T} serves as a tool for calculation of the starting template amount in each sample. The ΔC_{T} value describes the difference between the C_{T} value of the target gene and the C_{T} value of the corresponding endogenous reference gene. When applying the comparative ΔΔC_{T} method, the preparation of a standard curve is only required to determine amplification efficiencies of the target and the endogenous reference gene in an initial experiment. The comparative ΔΔC_{T} method for relative quantification is a standard method known in the art and is, e.g., described in detail in Livak and Schmittgen, 2001.

In another aspect, the present invention provides a kit, comprising a first and a second polynucleotide as defined in the context of the present invention, a set of dNTPs, a reverse transcriptase enzyme, and a detection moiety, preferably one or more hydrolysis probes specific for one or more different RNA molecules.

In a preferred embodiment of said aspect, the kit further comprises (i) an enzyme with Uracil-DNA-glycosylase (UNG) activity, and/or (ii) a first and/or a second primer as defined in the context of the present invention.

In a further aspect, the present invention relates to the use of a kit according to the present invention for the detection of an RNA molecule, preferably for the detection of one or more RNA molecule(s) as defined in the context of the present invention.

The following Figures and Examples are intended to illustrate various embodiments of the present invention. As such, the specific modifications discussed therein are not to be understood as limitations of the scope of the invention. It will be apparent to the person skilled in the art that various equivalents, changes, and modifications may be made without departing from the scope of the invention, and it is thus to be understood that such equivalent embodiments are to be included herein.

### FIGURES

**Fig. 1** illustrates the principle of method I for the detection of mature and precursor miRNA using a first polynucleotide with sequence-specificity to the target RNA molecule (comprising a first primer binding site, here designated universal primer binding site B = UPB). The second polynucleotide (dU helper oligo, comprising a second primer binding site = universal primer binding site A, UPA) is digested before the PCR reaction using the enzyme UNG. PCR amplification of the extension reaction product is carried out by the use of a first and a second primer (i.e. UPA and UPB, respectively). The detection of the two amplification products is carried out in the presence of two different hydrolysis probes which are complementary to the mature and precursor first strand cDNA, respectively, and which are labeled with different donor/acceptor moieties (i.e. FAM/BHQ2 and HEX/BHQ2).
**Fig. 2** illustrates the principle of method II for the detection of miRNA using a first polynucleotide complementary to a poly (A) tail of mature and precursor miRNA and a degradable second polynucleotide (dU helper oligo). Here, the 3'-ends of the target RNA molecules are first elongated by enzymatic reaction of a poly(A)-polymerase, thus generating a poly(A) tail to which the first polynucleotide is hybridizing. The subsequent steps correspond to the steps of method I as illustrated in Fig. 1.
**Figs. 3-6**
   Real Time PCR amplification plots showing increase in fluorescence measured for FAM over 47 amplification cycles
**Fig. 3** shows the detection of a synthetic miR-21 RNA oligonucleotide molecule (50 fg) by means of real-time RT-PCR fluorescent readout using a sequence-specific first polynucleotide complementary to mature miR-21 RNA (method I).
**Fig. 4** shows the detection of endogenously expressed miR-21 RNA from lung carcinoma and normal tissue by means of real-time RT-PCR fluorescent readout using a sequence-specific first polynucleotide complementary to mature miR-21 RNA (method I) for reverse transcription.
**Fig. 5** shows the simultaneous detection of mature and precursor miR-21 RNA (with synthetic oligonucleotides as input material) by means of real-time RT-PCR fluorescent readout (method I) using sequence-specific first polynucleotides complementary to the mature and the precursor forms of miR-21 RNA for reverse transcription, respectively.
**Fig. 6** shows the simultaneous detection of mature and precursor miR-21 RNA (with synthetic oligonucleotides as input material) by means of real-time RT-PCR fluorescent readout using a first polynucleotide complementary to the poly(A) tail (method II) for reverse transcription.

### EXAMPLES

### Example 1

### Method I

Detection of miRNA with specific RT-primer (first polynucleotide) and dU DNA helper oligos (second polynucleotide) for first strand cDNA elongation

### Reverse transcription

*1. miRNA & RT-primer denaturation:* 4.5 µl containing miRNA and 60 nM (final concentration in 10 µl RT reaction) specific RT-primer are denaturated at 65 °C for 10 min and then immediately cooled to 4 °C or on ice.
*2. Addition of reaction components for reverse transcription:* 4.5 µl denaturated miRNA/RT primer + 2 µl 5x RT buffer + 0,25 µl RNase Inhibitor + 1 µl dNTP mix + 0,25 µl RT enzyme + 2 µl PCR grade water = 10 µl RT reaction (see pack insert of Transcriptor First Strand cDNA Synthesis Kit, Roche, Germany)
*3. Reverse transcription:* 55 °C 5 min reverse transcription, 85 °C 5 min inactivation RT enzyme, 4 °C cooling
*4. Addition of dU DNA polynucleotide and additional RT enzyme:* + 1 µl 60 nM dU DNA oligo + 0,5 µl 1:4 diluted RT enzyme (Transcriptor First Strand cDNA Synthesis Kit, Roche, Germany)
*5. Elongation of first strand cDNA:* 55 °C 5 min reverse transcription, 85 °C 5 min inactivation RT enzyme, 4 °C cooling or storage at -20°C

### dU DNA polynucleotide degradation, amplification and detection

*6. dU DNA polynucleotide degradation and subsequent PCR reaction:* 1 µl cDNA from reverse transcription + 1 x Taqman RNA AMP Kit RNA Mix + 0,5 µM universal PCR primer A + 0,5 µM universal PCR primer B + 0,2 µM mi21matu-Probe + 2,8 mM MgAc (20 µl PCR reaction)
*7. PCR program on LC480 PCR cycler (Roche Diagnostics GmbH, Germany)* Reaction volume: 20 µl; Detection format: dual color hydrolysis probe;
   Cycles for amplification: 47, 95 °C 15 s, 60 °C 25 s; Cooling 40 °C 30 min

**Table 1: Polynucleotide sequences**

| **Polynucleotide name** | **Sequence** |
|---|---|
| mature specific RT-primer | gccacccacgagccacgcatcaacat (SEQ ID No.: 1) |
| precursor specific RT-primer | gccacccacgagccacgcatgtcaga (SEQ ID No.: 2) |
| dU DNA oligo : mi21-dU-prec-helper-UPB | gccuugccuaguccucucaguaauuauauuugucggguagcuuaucag (SEQ ID No.: 3) |
| Universal PCR primer A | gccacccacgagccacgca (SEQ ID No.: 4) |
| Universal PCR primer B | gccttgcctagtcctctcag (SEQ ID No.: 5) |
| mi21matu-Probe | FAM-tcgggtagcttatcagactgatgttga-BHQ2 (SEQ ID No.: 6) |
| mi21prec-Probe | HEX-cagcccatcgactggtgttgccatgag-BHQ2 (SEQ ID No.: 7) |

### Example Ia:

Detection of 50 fg mature miR-21 as synthetic oligo using mature specific RT-primer for reverse transcription and degradable dU DNA oligos for cDNA elongation. The result is shown in Fig. 3. Conclusion: Mature miR-21 as synthetic polynucleotide can be detected using mature specific RT-primer in reverse transcription and degradable dU DNA oligos for cDNA elongation.

### Example Ib:

Detection of mature miR-21 in miRNA isolated from K562 cell line using mature specific RT-primer for reverse transcription and degradable dU DNA oligos for cDNA elongation. Conclusion: Mature miR-21 in miRNA from K562 cell line can be detected using mature specific RT-primer in reverse transcription and degradable dU-DNA oligos for cDNA elongation.

### Example Ic:

Detection of mature miR-21 in miRNA isolated from lung carcinoma/normal tissue FFPET material using mature specific RT-primer for reverse transcription and degradable dU DNA oligos for cDNA elongation. The result is shown in Fig. 4. Conclusion: Mature miR-21 in miRNA isolated from lung carcinoma/normal tissue FFPET material can be detected using mature specific RT-primer in reverse transcription and degradable dU DNA oligos for cDNA elongation.

### Example Id:

Detection of precursor miR-21 (input material: synthetic oligoribonucleotide) using precursor specific RT-primer for reverse transcription and degradable dU DNA oligos for cDNA elongation. Conclusion: Precursor miR-21 provided in form of a synthetic oligoribonucleotide can be detected using precursor specific RT-primer in reverse transcription and degradable dU DNA oligos for cDNA elongation.

### Example Ie:

Simultaneous detection of mature and precursor miR-21 (input material: synthetic oligonucleotides) using mature and precursor specific RT-primer for reverse transcription and degradable dU DNA oligos for cDNA elongation. The result is shown in Fig. 5. Conclusion: Mature and precursor miR-21 can be detected in the same reaction using mature and precursor specific RT-primer in reverse transcription and degradable dU DNA oligos for cDNA elongation. The detection of precursor in a background of mature miR-21 is still possible down to 1% precursor.

### Example 2

### Method II

Detection of miRNA with (anchored) oligo-dT RT-primer (first polynucleotide) and dU DNA helper oligos (second polynucleotide) for first strand cDNA elongation

### PolyA tailing and miRNA isolation

*1. PolyA tailing:* according to pack insert Poly(A) Tailing Kit (Ambion Inc., Austin, TX, USA)
*2. Isolation of poly-adenylated miRNA:* according to 1-column protocol for the isolation of total RNA containing small RNA of pack insert High Pure miRNA Isolation Kit (Roche Diagnostics GmbH, Germany). Only change: equal amounts of buffers BB and BE were used like recommended for isolation using cell/tissue lysates.

### Reverse transcription

*3. miRNA & RT-primer denaturation:* 4.5 µl containing miRNA and 200 nM (final concentration in 10 µl of RT reaction volume) of anchored oligo-dT RT-primer are denaturated at 65 °C for 10 min and then immediately cooled to 4 °C or on ice.
*4. Addition of reaction components for reverse transcription:* 4.5 µl denaturated miRNA/RT primer + 2 µl 5x RT buffer + 0.25 µl RNase Inhibitor + 1 µl dNTP mix + 0.25 µl RT enzyme + 2 µl PCR grade water = 10 µl RT reaction (pack insert Transcriptor First Strand cDNA Synthesis Kit, Roche Diagnostics GmbH, Germany)
*5. Reverse transcription and elongation of first strand DNA:* 55 °C 5 min reverse transcription; 85 °C 5 min inactivation RT enzyme; 55 °C 12 min reverse transcription, immediately after reaching 55 °C addition of + 1µl 100 nM dU DNA oligo + 0,5 µl 1:4 diluted RT enzyme (Transcriptor First Strand cDNA Synthesis Kit, Roche); 85 °C 5 min inactivation RT enzyme; 4 °C cooling or storage at -20°C

### dU DNA oligo degradation, amplification and detection

*6. dU DNA oligo degradation and subsequent PCR reaction:* 1 µl cDNA from reverse transcription + 1 x Taqman RNA AMP Kit RNA Mix + 0.5 µM universal PCR primer A + 0.5 µM universal PCR primer B + 0.2 µM mi21matu-Probe + 2.8 mM MgAc (20 µl PCR reaction)
*7. PCR program on LC480 PCR cycler (Roche Diagnostics GmbH, Germany)*
   Reaction volume: 20 µl; Detection format: dual color hydrolysis probe; cycles for amplification: 47, 95 °C 15 s, 60 °C 25 s; Cooling 40 °C 30 min

**Table 2: Polynucleotide sequences**

| **Polynucleotide name** | **Sequence** |
|---|---|
| mature anchored RT-primer: mi21matu-rev-UPA | gcctccctcgcgccatcagtttttttttttttttttcaa (SEQ ID No.: 8) |
| precursor anchored RT-primer: mi21 prec-rev-UPA II | gcctccctcgcgccatcagtttttttttttttttttgtc (SEQ ID No.: 9) |
| dU DNA oligo : Mi21-dU-helper-UPB IIg | gccuugccuaguccucucaguaauuauauuugucgguagcuuaucagacugau gu-phosphat (SEQ ID No.: 10) |
| Universal PCR primer A | gcctccctcgcgccatcag (SEQ ID No.: 11) |
| Universal PCR primer B | gccttgcctagtcctctcag (SEQ ID No.: 5) |
| Mi21matu-Probe | FAM-tcgggtagcttatcagactgatgttga-BHQ2 (SEQ ID No.: 6) |
| Mi21prec-Probe | HEX-cagcccatcgactggtgttgccatgag-BHQ2 (SEQ ID No.: 7) |

### Example IIa:

Detection of 5ng mature miR-21 as synthetic oligo using (mature anchored) oligo-dT RT-primer for reverse transcription and degradable dU DNA oligos for cDNA elongation. Conclusion: Mature miR-21 as synthetic oligo can be detected using (mature anchored) oligo-dT RT-primer in reverse transcription and degradable dU DNA oligos for cDNA elongation. The negative control shows some dimer formation which does not affect the miRNA detection (as long as the miRNA curves appear earlier than the dimer curves).

### Example IIb:

Detection of mature miR-21 in miRNA isolated from K562 cell line using (mature anchored) oligo-dT RT-primer for reverse transcription and degradable dU DNA oligos for cDNA elongation. Conclusion: Mature miR-21 in miRNA from K562 cell line can be detected using (mature anchored) oligo-dT RT-primer in reverse transcription and degradable dU DNA oligos for cDNA elongation. The negative control shows some dimer formation which does not affect the miRNA detection (as long as the miRNA curves appear earlier than the dimer curves).

### Example IIc:

Detection of mature miR-21 in miRNA isolated from colon carcinoma FFPET material using (mature anchored) oligo-dT RT-primer for reverse transcription and degradable dU DNA oligos for cDNA elongation. Conclusion: Mature miR-21 in miRNA isolated from colon carcinoma FFPET material can be detected using (mature anchored) oligo-dT RT-primer in reverse transcription and degradable dU DNA oligos for cDNA elongation. The negative control shows some dimer formation which does not affect the miRNA detection (as long as the miRNA curves appear earlier than the dimer curves).

### Example IId:

Detection of precursor miR-21 as synthetic oligo using (precursor anchored) oligo-dT RT-primer for reverse transcription and degradable dU DNA oligos for cDNA elongation. Conclusion: Precursor miR-21 as synthetic oligo can be detected using (precursor anchored) oligo-dT RT-primer in reverse transcription and degradable dU DNA oligos for cDNA elongation. The negative control in the mature channel shows some dimer formation which does not affect the miRNA detection (as long as the miRNA curves appear earlier than the dimer curves).

### Example IIe:

Simultaneous detection of mature and precursor miR-21 (as synthetic oligos) using (mature and precursor anchored) oligo-dT RT-primer for reverse transcription and degradable dU DNA oligos for cDNA elongation. Conclusion: Mature and precursor miR-21 (as synthetic oligos) can be detected in the same reaction using (mature and precursor anchored) oligo-dT RT-primer in reverse transcription and degradable dU DNA oligos for cDNA elongation. The negative control in the mature channel shows some dimer formation which does not affect the miRNA detection (as long as the miRNA curves appear earlier than the dimer curves).

### References

Bartel D.P (2009), Cell, 136: 215-33.
Gaur A., Jewell D.A., Liang Y., Ridzon D. et al. (2007), Cancer Res. 67: 2456-2468.
Kumar M.S., Lu J., Mercer K.L., Golub T.R., Jacks T. (2007), Nat. Genetics 39: 673-677.
Livak K.J. and Schmittgen T.D. (2001), Methods 25: 402-408.
Lu J., Getz G., Miska E.A. et al. (2005), Nature 435: 834-838.
Sassen S., Miska E.A., Caldas C. (2008), Virchows Arch 452: 1-10.
Schmittgen T.D., Lee E.J., Jiang J., Sarkar et al. (2008), Methods 44(1): 31-38.

## Claims

1. A method for the detection of a RNA molecule, said method comprising the steps of
a) providing a sample containing the RNA molecule;
b) hybridizing to said RNA molecule a first polynucleotide comprising a first primer binding site;
c) extending the first polynucleotide by reverse transcribing the sequence of the RNA molecule to generate a first strand cDNA;
d) hybridizing a second polynucleotide to the first strand cDNA, **characterized in that** said second polynucleotide is a 3'-non-extendable oligonucleotide comprising
(i) a 3'-portion complementary to a portion of the first strand cDNA, and
(ii) a 5'-overhang comprising a sequence of a second primer binding site;
e) extending the first strand cDNA to generate an extension reaction product comprising a sequence complementary to the second primer binding site;
f) amplifying the extension reaction product by means of polymerase chain reaction in the presence of a detection moiety using a first primer complementary to the first primer binding site and a second primer complementary to the second primer binding site; and
g) detecting the amplification product by means of real-time fluorescence readout.

2. The method of claim 1, **characterized in that** the RNA molecule has a length of from 15 to 200 nucleotides, preferably of from 20 to 100 nucleotides.

3. The method of claim 1 or 2, **characterized in that** the RNA molecule is selected from the group consisting of mature miRNA (miRNA), precursor miRNA (pre-miRNA), primary miRNA precursor (pri-miRNA), small interfering RNA (siRNA), piRNA (piwi-interacting RNA), precursor piRNA, and short hairpin RNA (shRNA).

4. The method of any of claims 1 to 3, **characterized in that** the RNA molecule of step a) is extended at the 3'-terminus by a polynucleotide tail, preferably via enzymatic reaction of a poly(A) polymerase, a terminal transferase, or a ligase, more preferably via enzymatic reaction of a poly A polymerase.

5. The method of any of claims 1 to 4, **characterized in that** the first polynucleotide is complementary to the sequence of the RNA molecule, complementary to the polynucleotide tail, or complementary to both.

6. The method of any of claims 1 to 5, **characterized in that** the second polynucleotide is a degradable polynucleotide.

7. The method of claim 6, **characterized in that** the second polynucleotide comprises dU-nucleotide residues and is digested after step e) by enzymatic reaction of Uracil-DNA-glycosylase (UNG).

8. The method of any of claims 1 to 7, **characterized in that** the second polynucleotide has a blocked 3'-terminus in form of a 3'-terminal phosphate.

9. The method of any of claims 1 to 8, **characterized in that** the detection moiety in step f) is an intercalating dye.

10. The method of any of claims 1 to 8, **characterized in that** the detection moiety in step f) is a hydrolysis probe.

11. The method of claim 10, **characterized in that** at least two different extension reaction products are amplified in step f).

12. The method of claim 11, **characterized in that** the amplification in step f) is carried out in the presence of at least two hydrolysis probes, wherein at least one of said hydrolysis probes is specific for a specific species of RNA molecules, and wherein the probes comprise different sets of donor/acceptor moieties.

13. The method of claim 12, **characterized in that** the specific species of RNA molecules are derived from one precursor molecule, preferably having different lenghts.

14. The method of any of claims 11 to 13, **characterized in that** the ratio between the different species of RNA molecules is determined by the detection of different amplification products, preferably by the detection of different amplification products with distinguishable fluorescent readout.

15. A kit, comprising:
a first and a second polynucleotide as defined in any of the preceding claims,
a set of dNTPs,
a reverse transcriptase enzyme, and
a detection moiety, preferably one or more hydrolysis probes specific for one or more different RNA molecules.

16. The kit of claim 15, further comprising (i) an enzyme with Uracil-DNA-glycosylase (UNG) activity, and/or (ii) a first and a second primer as defined in any of the preceding claims.

17. Use of the kit according to claim 15 or 16 for the detection of an RNA molecule, preferably for the detection of one or more RNA molecule(s) as defined in any of claims 2-3 and 11-14.
